# FASCICULE DE BREVET EUROPEEN

(11) **EP 3 174 979 B1**
(45) Date de publication et mention de la délivrance du brevet: **19.02.2020**
(21) Numéro de dépôt: 15742261.9
(22) Date de dépôt: 27.07.2015
(51) Int. Cl.: C12N 9/42, C12N 1/06, C12N 1/14, C12R 1/885

(54) **PROCEDE DE PRODUCTION D'UN COCKTAIL ENZYMATIQUE A PARTIR DE MOÛT DE CHAMPIGNON**
VERFAHREN ZUR HERSTELLUNG EINES ENZYMATISCHEN GEMISCHS AUS PILZSCHIMMEL
METHOD FOR PRODUCING AN ENZYMATIC COCKTAIL FROM FUNGAL MUST

(30) Priorité: 30.07.2014 FR 1457358
(43) Date de publication de la demande: 07.06.2017
(73) Titulaire: IFP Energies nouvelles, 92500 Rueil-Malmaison (FR)
(72) Inventeur: BEN CHAABANE, Fadhel, F-75020 Paris (FR); JOURDIER, Etienne, F-92420 Vaucresson (FR); COHEN, Celine, F-75017 Paris (FR); CHAUSSEPIED, Bernard, F-28130 Hanches (FR)
(74) Mandataire: IFP Energies nouvelles
(86) Numéro de dépôt international: PCT/EP2015/067139
(87) Numéro de publication internationale: WO 2016/016182

(56) Documents cités:
- WO-A2-2005/100582
- S ROUSSOS ET AL: "HYDROLYSE DE LA CELLULOSE PAR LES MOISISSURES II. PRODUCTION DE CELLULASE DE TRICHODERMA HARZIANUM PAR FERMENTATION EN MILIEU LIQUIDE", ANNALES DE MICROBIOLOGIE (INST. PASTEUR), vol. 133 B, 1 janvier 1982 (1982-01-01), pages 465-474, XP055177608, ISSN: 0300-5410
- PITSON ET AL: "Induction and carbon source control of extracellular beta-glucosidase production in Acremonium persicinum", MYCOLOGICAL RESEARCH, ELSEVIER, GB, vol. 103, no. 2, 1 février 1999 (1999-02-01), pages 161-167, XP022451476, ISSN: 0953-7562, DOI: 10.1017/S0953756298006777
- RAO M ET AL: "LAMINARINASE FROM PENICILLIUM-FUNICULOSUM AND ITS ROLE IN RELEASE OF BETA GLUCOSIDASE", BIOTECHNOLOGY AND APPLIED BIOCHEMISTRY, ACADEMIC PRESS, US, vol. 13, no. 2, 1 janvier 1991 (1991-01-01), pages 277-285, XP008177670, ISSN: 0885-4513, DOI: 10.1111/J.1470-8744.1991.TB00156.X [extrait le 2010-12-23]
- COPA-PATINO J ET AL: "Purification and properties of a beta-glucosidase from Penicillium oxalicum autolysates", FEMS MICROBIOLOGY LETTERS, WILEY-BLACKWELL PUBLISHING LTD, GB, vol. 67, no. 1-2, 15 janvier 1990 (1990-01-15), pages 191-196, XP023918497, ISSN: 0378-1097 [extrait le 1990-01-15]
- GAIKWAD ET AL: "Localization and release of beta-glucosidase in the thermophilic and cellulolytic fungus, Sporotrichum thermophile", EXPERIMENTAL MYCOLOGY, ACADEMIC PRESS, LONDON, GB, vol. 18, no. 4, 1 décembre 1994 (1994-12-01), pages 300-310, XP022112073, ISSN: 0147-5975, DOI: 10.1016/S0147-5975(06)80003-4
- S.N MUKHOPADHYAY ET AL: "Increased production of cellulase of Trichoderma sp. by pH cycling and temperature profiling", BIOTECHNOLOGY AND BIOENGINEERING, vol. 22, no. 11, 1 janvier 1980 (1980-01-01), pages 2237-2250, XP055177765, ISSN: 0006-3592
- KUBICEK C P: "BETA GLUCOSIDASE EXCRETION BY TRICHODERMA-PSEUDOKONINGII CORRELATION WITH CELL WALL BOUND BETA-1.3 GLUCANASE EC-3.2.1.6 ACTIVITIES", ARCHIVES OF MICROBIOLOGY, vol. 132, no. 4, 1982, pages 349-354, XP008175515, ISSN: 0302-8933
- EGYHAZI A ET AL: "Examination of cellulose enzyme production by Trichoderma Reesei rut C30 using supercritical carbon dioxide cell disruption.", CHEMICAL AND BIOCHEMICAL ENGINEERING QUARTERLY, vol. 18, no. 3, 1 janvier 2004 (2004-01-01), pages 257-261, XP055177583, ISSN: 0352-6568
- MARTINEZ M J ET AL: "beta-Glucosidase from the cellulolytic system of Alternaria alternata autolyzed cultures", FEMS MICROBIOLOGY LETTERS, WILEY-BLACKWELL PUBLISHING LTD, GB, vol. 55, no. 3, 15 octobre 1988 (1988-10-15), pages 263-267, XP023971301, ISSN: 0378-1097 [extrait le 1988-10-15]
- UMILE C ET AL: "A constitutive, plasma-membrane bound beta-glucosidase in Trichoderma reesei", FEMS MICROBIOLOGY LETTERS, WILEY-BLACKWELL PUBLISHING LTD, GB, vol. 34, no. 3, 1 mai 1986 (1986-05-01), pages 291-295, XP023920331, ISSN: 0378-1097 [extrait le 1986-05-01]
- KOVACS K ET AL: "Comparative enzymatic hydrolysis of pretreated spruce by supernatants, whole fermentation broths and washed mycelia of Trichoderma reesei and Trichoderma atroviride", BIORESOURCE TECHNOLOGY, ELSEVIER BV, GB, vol. 100, no. 3, 1 February 2009 (2009-02-01), pages 1350-1357, XP025645373, ISSN: 0960-8524, DOI: 10.1016/J.BIORTECH.2008.08.006 [retrieved on 2008-09-14]

## Description

La présente invention concerne l'amélioration de la production d'enzymes cellulolytiques et/ou hémicellulolytiques, notamment dans le cadre de la production d'éthanol à partir de matériaux cellulosiques ou ligno-cellulosiques.

Ces derniers sont produits à partir de biomasse lignocellulosique et, en ce qui concerne l'usage des terres agricoles pour la production de biocarburants, posent moins de problèmes de concurrence avec l'alimentaire, que les procédés dits de première génération produits à partir de canne à sucre, maïs, blé, betterave...

Les différentes études technico-économiques démontrent que la réduction du coût des cellulases est un des points-clés des procédés de production biologique d'éthanol à partir des matières premières lignocellulosiques.
A l'heure actuelle, les cellulases industrielles sont principalement produites par un champignon filamenteux, *Trichoderma reesei,* en raison de son fort pouvoir de sécrétion. Dans les procédés classiques de production de cellulases, celles-ci sont récupérées dans le surnageant de culture par plusieurs étapes de séparation.
Le moût de champignon (partie solide récupéré après filtration) n'est pas valorisé. Il est considéré comme déchet.
De plus, le cocktail enzymatique produit par les souches hyper-productrices de *Trichoderma reesei* est pauvre en activité β-glucosidase.

Des brevets ont proposé des procédés pour améliorer la souche productrice de plusieurs façons : en sur-exprimant le gène (tel que la demande de brevet EP2082054 concernant la surexpression d'une β-glucosidase), ou en clonant des β-glucosidases plus efficaces provenant d'autres micro-organismes (tel que la demande de brevet WO2013115305 qui décrit le clonage du gène β-glucosidase d'Aspergillus chez *Trichoderma reesei*) ou encore en créant des nouveaux gènes plus efficaces à partir de différents gènes de β-glucosidase (tel que la demande de brevet WO 2010029259 qui décrit la production de variants de β-glucosidase à activité améliorée par L-Shuffling).

On sait également par la demande de brevet WO 11079048 que, dans un procédé SSF (hydrolyse et fermentation simultanées), l'augmentation de l'activité β-xylosidase a un effet bénéfique sur l'hydrolyse enzymatique puisqu'elle permet de réduire la dose d'enzymes utilisée. Elle permet aussi l'hydrolyser les alkyl xylosides. L'invention propose un procédé de production d'un cocktail enzymatique et une installation convenant à la mise en œuvre du procédé selon l'invention, cocktail qui permet d'améliorer de façon significative, voire drastique, les activités β-glucosidase et β-xylosidase.

En effet, on a constaté, de façon surprenante, que la mise en œuvre du procédé selon l'invention conduit à une forte amélioration de l'activité spécifique β-glucosidase, FPase et β-xylosidase. L'activité β-glucosidase peut être entre 3 et dix fois supérieure à celle mesurée en fin d'une production d'enzymes dans les conditions normales ; celle de la β-xylosidase, 3 fois supérieure.
De ce fait, le moût de champignon séparé des enzymes lors d'une étape classique de production d'enzymes est valorisé, ce moût représente 10-20% en masse par rapport à la masse totale de la culture.

Plus précisément, l'invention concerne un procédé pour augmenter le taux de β-glucosidase et/ou de β-xylosidase d'un cocktail enzymatique à partir d'un microorganisme cellulolytique produisant des cellulases et/ou des hémi-cellulases, comprenant :
- une étape de production d'enzymes avec obtention d'un milieu contenant des enzymes et un moût de microorganisme, ledit moût est séparé ou non séparé du liquide contenant lesdites enzymes,
- une étape de refroidissement dudit moût à une température comprise entre 4 et 20°C, qui est une température inférieure à la température de l'étape de production d'enzymes et pendant un temps compris entre 12 et 90 h tel que :
- la concentration en β-glucosidase ou β-xylosidase du liquide issu de l'étape de refroidissement est supérieure à celle du liquide issu de l'étape de production d'enzymes,
   et/ou
- le ratio du volume du solide/ volume total est inférieur audit ratio pour l'étape de production d'enzymes,
- et il est obtenu à l'issue de l'étape de refroidissement un cocktail enzymatique, le microorganisme appartenant au genre *Trichoderma.*

Avantageusement, la température de l'étape de refroidissement est comprise entre 4 et 20°C, et de préférence entre 4°C et 18°C.
De préférence, l'étape de refroidissement est réalisée à un pH compris entre 3,5 et 5,5, et de préférence supérieur à 4.
De façon préférée, l'étape de refroidissement est réalisée dans une atmosphère appauvrie en oxygène, de préférence en atmosphère anaérobie.
Généralement, il est injecté un gaz neutre, par exemple de l'azote ou du gaz carbonique.
Le microorganisme appartient au genre *Trichoderma,* en particulier *Trichoderma reesei,* et de préférence il s'agit de la souche CL847.

Après l'étape de refroidissement, le cocktail enzymatique issu du moût est séparé et il est obtenu un moût résiduel.
Généralement, la séparation a lieu au moyen d'au moins une centrifugation ou filtration/ pressage ou microfiltration, éventuellement précédée d'une décantation. Les enzymes du liquide séparé peuvent être concentrées, par exemple par ultrafiltration.

Le microorganisme utilisé est choisi parmi les champignons cellulolytiques ou d'autres microorganismes modifiés. De façon préférée, le microorganisme cellulolytique appartient au genre *Trichoderma* qui produit notamment les cellulases et les hémicellulases adaptées à l'hydrolyse totale de la cellulose et des hémicelluloses.

Les souches industrielles utilisées appartiennent de façon préférée, à l'espèce *Trichoderma reesei,* le champignon a été généralement modifié pour améliorer la production d'enzymes cellulolytiques et/ou hémicellulolytiques par des procédés de mutation-sélection (mutagénèse alléatoire), comme par exemple la souche IFP CL847 (brevet français FR-B-2 555 803). Ces souches sont bien connues de l'homme du métier.

Le procédé selon l'invention comporte une étape de production d'enzymes avec obtention d'un milieu contenant des enzymes et un moût de microorganisme, et avec une éventuelle séparation dudit moût du liquide contenant les enzymes.

Les souches sont cultivées en fermenteur agité et aéré dans des conditions compatibles avec leur croissance et la production des enzymes, ces conditions sont connues de l'homme du métier. Une source de carbone pour la croissance et une source inductrice pour la production d'enzymes sont introduites. La source de carbone peut être un sucre soluble industriel, par exemple le glucose, le lactose ou le xylose, ou un extrait de la fraction hémicellulosique sous forme de monomères provenant de la biomasse prétraitée. La source de carbone inductrice peut être choisie parmi le lactose, le cellobiose, le sophorose, la cellulose. Le résidu d'hydrolyse ou le matériel lignocellulosique prétraité peuvent aussi être utilisés comme source de carbone pour la croissance du microorganisme et l'induction du système d'expression. Cette dernière source de carbone est aussi utilisable par les souches améliorées génétiquement et notamment les souches recombinantes.

A l'issue de l'étape de production d'enzymes, le moût est séparé du liquide ou n'est pas séparé du liquide ou encore une partie seulement du moût peut être séparée. Le moût correspond au champignon, à la partie solide. Le liquide contient les enzymes. Le moût séparé (en totalité ou en partie) peut être dilué. De préférence le moût séparé est dilué par de l'eau.

On pourra adapter la séparation notamment en fonction des activités souhaitées.
La séparation peut être opérée par tout moyen connu de l'homme du métier. On citera de préférence au moins une centrifugation ou une filtration/pressage (filtre-presse) ou microfiltration. La centrifugation est éventuellement précédée d'une décantation. Une étape de concentration des enzymes, par exemple par ultrafiltration, peut être prévue.

Le procédé selon l'invention se poursuit par une étape de refroidissement dudit moût (séparé ou non) à une température inférieure à la température de l'étape de production d'enzymes et pendant un temps déterminé.

La température de l'étape de refroidissement est avantageusement comprise entre 4 et 20°C, et de préférence entre 4°C et 18°C.
De préférence, l'étape de refroidissement est réalisée à un pH compris entre 3,5 et 5,5, et de préférence supérieur à 4. Cet intervalle de pH est défini entre le pH 3 de début désactivation de la β-glucosidase et le pH 6 de possible sporulation du champignon.
De préférence, l'étape de refroidissement est réalisée dans une atmosphère appauvrie en oxygène, de préférence en atmosphère anaérobie. Généralement, il est injecté un gaz neutre, par exemple de l'azote ou du gaz carbonique. On a observé un rendement amélioré en atmosphère appauvrie en oxygène, et les meilleurs rendements sont en atmosphère anaérobie. En présence d'oxygène le champignon peut consommer à nouveau les enzymes.
Généralement, un refroidissement de préférence de 24 à 72h est suffisant, et généralement autour de 48h.

Pendant cette étape de refroidissement, une réaction d'autolyse du champignon présent dans le moût s'installe, autolyse générée par les enzymes. Celles-ci sont présentes soit dans le liquide du moût non séparé soit ce sont celles qui sont encore enserrées dans le moût séparé et qui n'ont pas pu être séparées.

On remarque expérimentalement que, dans les conditions du procédé selon l'invention, les activités β-glucosidase et β-xylosidase augmentent de façon très significative et que le culot de biomasse diminue.

Pour optimiser le rendement en cocktail enzymatique, le temps de refroidissement peut être déterminé préalablement dans un essai laboratoire.
Avantageusement, le temps de refroidissement est contrôlé lors de la mise en œuvre du procédé, l'avancement de la réaction est suivi par des prélèvements.

Le temps de refroidissement est tel que
- l'activité en β-glucosidase ou β-xylosidase du liquide issu de l'étape de refroidissement est supérieure à celle du liquide issu de l'étape de production d'enzymes,
   et/ou
- le ratio du volume du solide/ volume total (culot de biomasse) est inférieur audit ratio pour l'étape de production d'enzymes.
Le substrat utilisé pour déterminer l'activité β-glucosidase ou aryl β-glucosidase est le p-nitrophenyl-β-D-glucopyranoside (PNPG). Il est clivé par la β-glucosidase qui libère le p-nitrophenol.
Une unité d'activité aryl β-glucosidase est définie comme la quantité d'enzyme nécessaire pour produire 1µmol de p-nitrophenol à partir de PNPG par minute et est exprimé en IU/mL.
Le substrat utilisé pour déterminer l'activité β-xylosidase est le p-nitrophenyl-β-D-xylopyranoside selon le même principe.
Ces paramètres sont déterminés à partir de mesures.

On a observé qu'au cours du procédé, la concentration en β-glucosidase augmente, atteint un maximum puis décroît. Dans le même temps, on a observé que le culot de biomasse décroît.
A titre d'exemple, on a pu constater que généralement la quantité de liquide libéré est équivalente à au moins 10%, et le plus souvent 30-40% du poids initial du moût séparé et que la concentration en protéines, mesurée en termes d'activités de β-glucosidase et β-xylosidase, a été multipliée par 3.
La détermination du temps de refroidissement à partir de ces informations est de la compétence de l'homme du métier.

Après l'étape de refroidissement, le cocktail enzymatique (partie liquide) peut être ou non séparé du solide résiduel, de préférence il est séparé.
Les moyens de séparation sont ceux décrits précédemment.

On a pu constater que cette séparation est plus difficile, plus délicate lorsque le moût n'a pas été séparé juste à la fin de la culture.

Aussi, de façon très préférée, on opère une séparation du moût avant l'étape de refroidissement, de préférence avec un filtre-presse. De préférence, on sépare au moins 95 % du moût, et de façon encore plus préférée, le moût est séparé en totalité du liquide. On comprend ici que « en totalité » se rapporte à la méthode de séparation utilisée.

De préférence, ledit cocktail enzymatique issu de l'étape de refroidissement est mélangé aux enzymes issues de l'étape de production d'enzymes.
Ledit cocktail est mélangé en totalité ou en partie.
Ledit cocktail, mélangé ou non, est utilisé en hydrolyse enzymatique.

Le moût résiduel peut être soumis à une nouvelle étape de refroidissement éventuellement mais de préférence, suivie d'une séparation d'un nouveau cocktail enzymatique.
Les conditions de ces étapes sont celles décrites précédemment.
On indique ici une étape de refroidissement supplémentaire, mais leur nombre n'est pas limité.
Ainsi, l'invention concerne également un procédé mettant en œuvre les étapes précédentes dans lequel ledit moût résiduel est soumis à une étape de refroidissement, et il est obtenu à l'issue de l'étape de refroidissement un cocktail enzymatique, séparé ou non du moût résiduel. De préférence, ledit cocktail est séparé du moût résiduel de ladite étape de refroidissement.

De préférence, les cocktails enzymatiques issus des étapes de refroidissement sont mélangés. De préférence, ils sont mélangés aux enzymes issues de l'étape de production d'enzymes.
De façon générale, on peut mélanger chacun des cocktails entre eux et/ou avec les enzymes issues de l'étape de production d'enzymes. La proportion de chacune des composantes du mélange est déterminée selon l'utilisation dudit mélange.
De préférence, on mélange la totalité des cocktails et des enzymes.

Au moins un cocktail, mélangé ou non, est utilisé en hydrolyse enzymatique.

L'invention sera mieux comprise à partir des figures 1 à 6. Les figures 7 à 13 se rapportent aux exemples.
La figure 1 décrit le mode préféré de réalisation avec une étape de séparation entre l'étape de production d'enzymes et l'étape de refroidissement. La figure 2 est une représentation sans séparation du moût. Dans les figures 3 et 4, il est ajouté une étape supplémentaire de refroidissement aux figures 1 et 2 respectivement.
La figure 5 représente un mode de réalisation combinant la présence ou non de séparation.
La figure 6 représente un mode de réalisation préféré de l'étape de séparation du moût avant et/ou après l'étape de refroidissement.

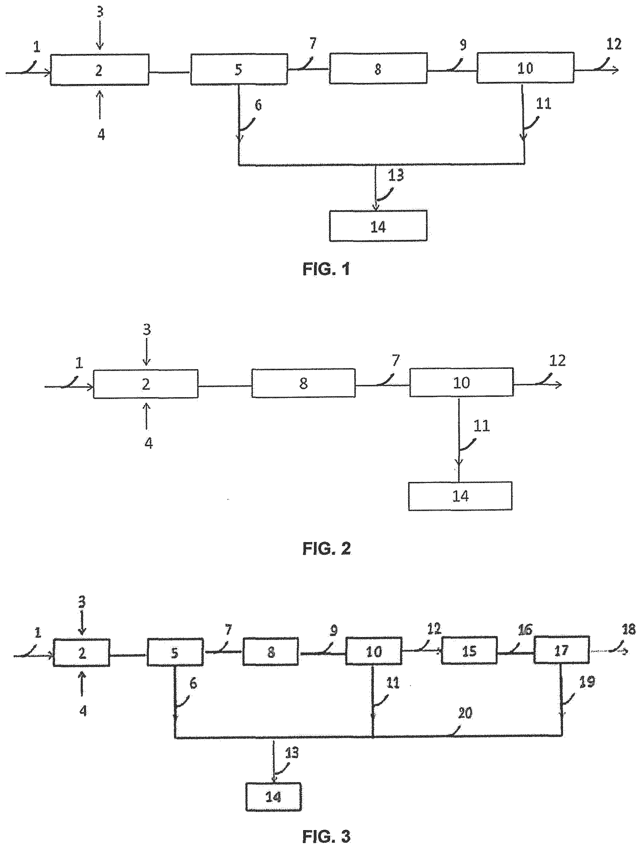

Selon la fig.1, une source de carbone et une source inductrice sont introduites (conduite 1) dans une étape de production d'enzymes (zone 2 de production d'enzymes), ainsi qu'un microorganisme cellulolytique produisant des cellulases et/ou des hémi-cellulases (conduite 3) et les nutriments nécessaires (conduite 4).
Le produit obtenu est séparé (zone 5 de séparation) en un liquide contenant les enzymes (conduite 6) et il est récupéré un moût (conduite 7) contenant le champignon et des enzymes enserrées.
Le moût est soumis à l'étape de refroidissement (zone 8 de refroidissement).

Dans un mode de réalisation, le liquide est soutiré de la zone de production d'enzymes (réacteur) et le moût reste dans ladite zone où il est refroidi.
Dans un autre mode que l'on préfère, le milieu de culture est soutiré de la zone de production d'enzymes et on le sépare dans un moyen de séparation (fitration/pressage, centrifugation...), de préférence après décantation et soutirage du liquide, pour obtenir le moût. Ce moût est introduit dans la zone de refroidissement qui peut être le réacteur de la zone de production d'enzymes (dans le cas d'un procédé discontinu) ou un autre réacteur (dans le cas d'un procédé en continu).
Le moût refroidi (conduite 9) est séparé (zone 10) en un liquide contenant le cocktail enzymatique (conduite 11) et un solide qui est le moût résiduel (conduite 12).
Les enzymes des flux des conduites 6 et 11 sont mélangés et sont envoyés (conduite 13) dans la zone d'hydrolyse enzymatique (14).

La figure 3 reprend ce schéma en ajoutant une étape de refroidissement.
Le moût résiduel (moût 12) est soumis à l'étape de refroidissement supplémentaire (zone 15 de refroidissement).
De la même façon que précédemment, le moût envoyé dans l'étape de refroidissement supplémentaire (zone 15) peut ne pas avoir été séparé dans la zone 10 après la première étape de refroidissement (zone 8).
Le moût refroidi (conduite 16) est séparé (zone 17) en un liquide contenant le cocktail enzymatique (conduite 19) et un solide qui est le moût résiduel (conduite 18).
Les enzymes du flux de la conduite 19 mélangées à celles des flux issus de l'étape de production d'enzymes (conduite 6), de la première étape de refroidissement (conduite 11) et sont envoyées (conduite 20) dans la zone d'hydrolyse enzymatique (14).

On reconnaîtra sur la figure 2 les références de la figure 1. L'étape de séparation (zone 5) après l'étape de production d'enzymes est supprimée.

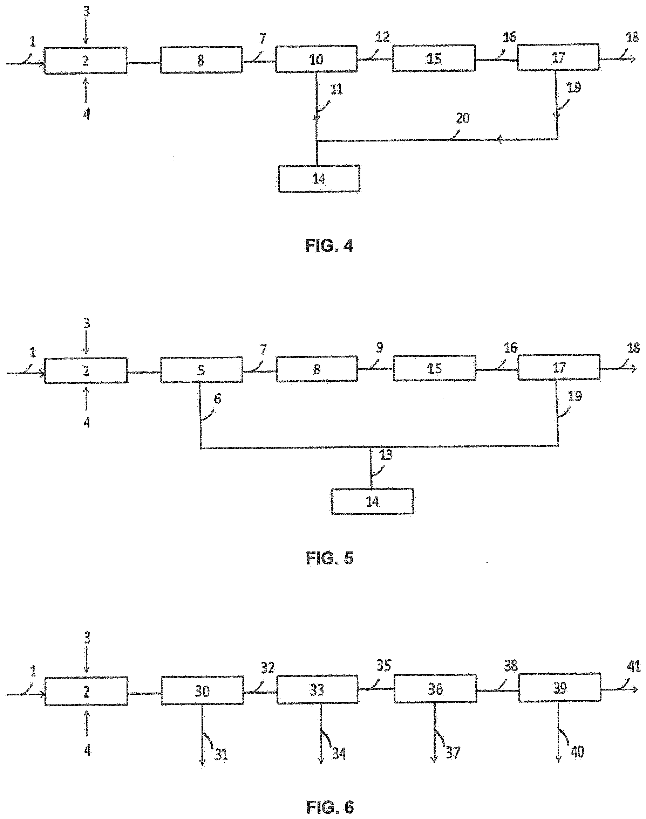

Il en est de même sur la figure 4 qui est le pendant de la figure 2 et qui inclut l'étape de refroidissement supplémentaire, les références relatives à cet ajout sont reprises de la figure 3.

On peut aussi combiner dans un schéma la présence d'une séparation et l'absence de l'autre séparation. Ceci est par exemple illustré fig.5. On y voit la présence de la séparation à l'issue de l'étape de production d'enzymes (zone 5), l'absence de séparation à l'issue de la première étape de refroidissement (zone 8) et avant l'étape de refroidissement supplémentaire, et la présence de séparation après l'étape de refroidissement supplémentaire (zone 17).

La figure 6 représente un mode de réalisation préféré de l'étape de séparation du moût avant et/ou après de l'étape de refroidissement.
Si on se rapporte à la figure 1, c'est un mode de réalisation de la zone 5 et/ ou de zone 10.
A l'issue de la zone 2 de production d'enzymes, le milieu de culture est séparé dans une étape de décantation (zone 30), le champignon se retrouve dans les boues décantées (conduite 31) et le jus trouble obtenu (conduite 32) passe dans une étape de centrifugation (zone de centrifugation 33). Il en ressort une crème (conduite 34) contenant le champignon et un jus clair (conduite 35). Pour achever la séparation, le jus clair passe dans une étape de microfiltration (zone 36), le rétentat (conduite 37) contient le champignon et le perméat (conduite 38) les enzymes.
Les différents flux contenant le champignon (boues, crème, rétentat) peuvent être mélangés et constituer le moût qui sera envoyé à l'étape de refroidissement ou constituer le moût résiduel, qui subira ou non une nouvelle étape de refroidissement. Afin de concentrer les enzymes, le perméat est passé dans une étape d'ultrafiltration. Un rétentat concentré d'enzymes (conduite 40) est alors obtenu, ainsi qu'un perméat (conduite 41) qui peut être réutilisé dans le procédé.
On notera que l'ensemble des étapes de décantation et centrifugation peut être remplacé par une filtration/pressage (filtre/presse).

### Exemples

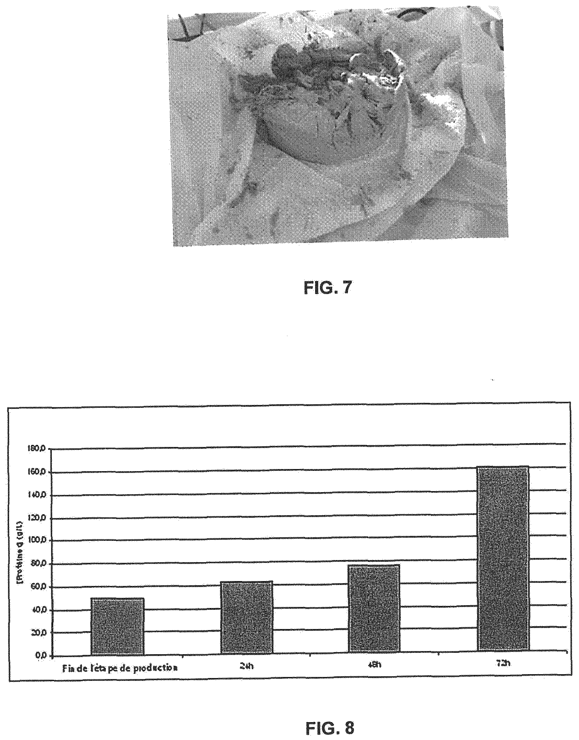

### Exemple 1 : avec les figures 7 à 9.

La figure 7 est une photo du moût de *Trichoderma reesei* CL847 après séparation et avant l'étape de refroidissement.
La figure 8 montre l'évolution de la concentration en protéines dans le surnageant de culture.

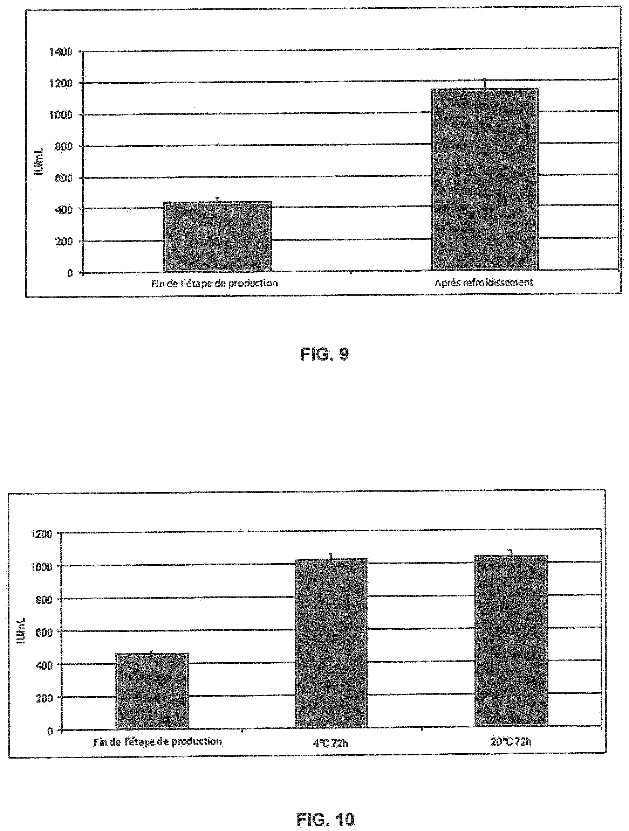

La figure 9 montre l'activité β-glucosidase mesurée en fin d'étape de production et après refroidissement durant 72h à 4°C du champignon.

La production de cellulases est effectuée en bioréacteur de 20 L (12L utiles) agité mécaniquement. Le milieu minéral a la composition suivante : KOH 1,66 g./L, H3P04 85 % 2 mL/L, (NH4)2SO4 2,8 g/L, MgSO4, 7 H2O 0,6 g/L, CaCL2 0,6 g/L, MnSO4 3,2 mg/L, ZnSO4, 7 H2O 2,8 mg/L, CoCl2 10 4,0 mg/L, FeS04, 7 H2O 10 mg/L, Corn Steep [en français liqueur de trempage de maïs séchée] 1,2 g/L, anti-mousse 0,5 mL/L.
Le bioréacteur contenant le milieu minéral est stérilisé à 120 °C pendant 20 minutes, la source carbonée glucose est stérilisée à part à 120°C pendant 20 minutes puis ajoutée stérilement dans le bioréacteur de façon à avoir une concentration finale de 30 g/L.
Le bioréacteur est ensemencé à 10% (v/v) avec une pré-culture liquide de la souche de *Trichoderma reesei* CL847. Le milieu minéral de la pré-culture, est identique à celui du bioréacteur mis à part l'addition de phtalate de potassium à 5 g/L pour tamponner le pH. La croissance du champignon en préculture est faite en utilisant le glucose comme substrat carboné, à la concentration de 30 g/L. La croissance de l'inoculum dure de 2 à 3 jours et est effectuée à 28 °C dans un incubateur agité. Le transfert vers le bioréacteur est réalisé si la concentration résiduelle en glucose est inférieure à 15 g/L.

L'expérience de production effectuée en bioréacteur comporte deux phases :
- une phase de croissance sur substrat carboné glucose (concentration initiale = 30 g/L) à une température de 27 °C et un pH de 4,8 (régulé par de l'ammoniaque 5.5 M). L'aération est de 0,5 vvm et l'agitation est augmentée entre 200 et 800 rpm en fonction de la p02 (pression en oxygène dissous), qui est maintenue supérieure à 30 %.
- une phase de production d'enzymes. Lorsque le substrat initial du fermenteur est épuisé, une solution de lactose à 250 g/L est injectée en continu au débit de 35 à 45 mg par g de cellules et par heure jusqu'à 164 heures. La température est abaissée à 25 °C et le pH à 4 jusqu'à la fin de la culture. Le pH est régulé par addition d'une solution d'ammoniaque à 5.5 N qui apporte l'azote nécessaire à la synthèse des protéines excrétées. La teneur en oxygène dissous est maintenue au-dessus de 15 à 20 % par action sur l'aération et l'agitation.
La production d'enzymes est suivie par le dosage des protéines extracellulaires par la méthode de Lowry et standard BSA, après séparation du mycélium par filtration ou de cellules formées). La concentration finale en protéines obtenue est égale à 45 g/L.

Après cette étape de production, nous avons procédé à une séparation du moût de champignons du surnageant de culture. Le moût a été pressé de façon à extraire un maximum de surnageant (figure7) et pesé. Celui-ci a été placé dans un récipient clos à 4°C. pendant 72h.
Des prélèvements du surnageant libéré suite à l'autolyse du champignon ont été effectués toutes les 24h jusqu'à 72h.
Le poids du liquide à la fin des 72h a été pesé. Il est équivalent à 30% du poids du moût. Les dosages des concentrations en protéines sont présentés sur la figure 8. On voit que la concentration est multipliée par 3 par rapport à la fin de la production d'enzymes ainsi que l'activité β-glucosidase (figure 9)

### Exemple2 : avec les figures 10 à 13.

La figure 10 montre l'activité β-glucosidase mesurée en fin de culture et après 72h d'autolyse à 4° et 20°C du champignon.

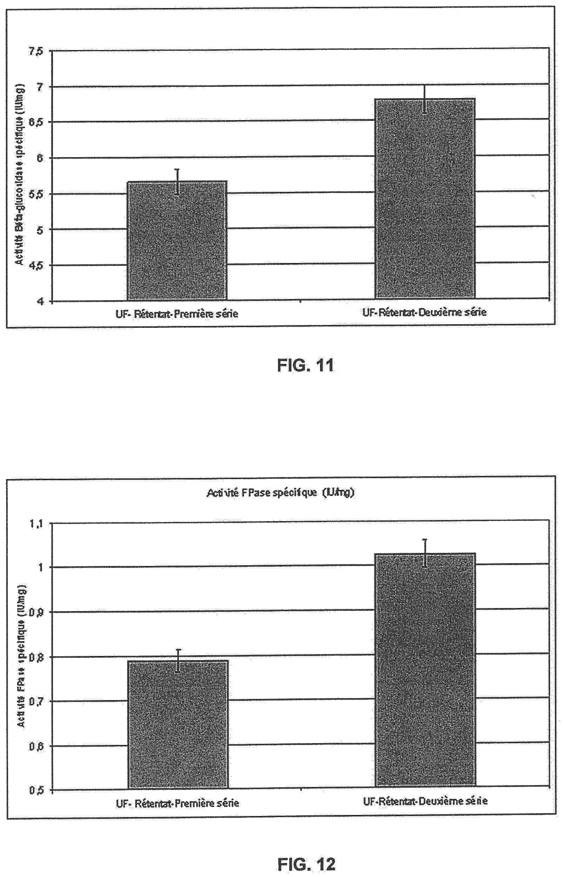

La figure 11 montre l'activitéβ-glucosidase spécifique (IU/mg) obtenue après les deux séries de séparation.
La figure 12 montre l'activité FPase spécifique (IU/mg) obtenue après les deux séries de séparation.

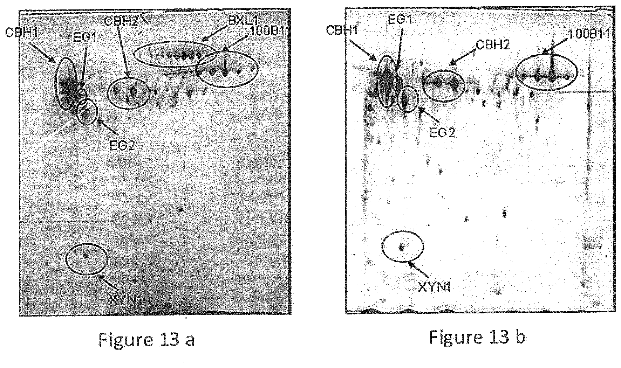

La figure 13 correspond à l'identification des enzymes dans l'étape de séparation.

Le procédé est mis en application dans un pilote de 6m³. Le champignon *Trichoderma reesei* CL847 est cultivé dans les mêmes conditions décrites dans l'exemple 1. A la fin de la phase de production les enzymes sont séparées en 4 étapes: une étape de décantation, une étape de centrifugation, une étape de microfiltration et une étape d'ultrafiltration.
Ces quatre étapes sont schématisées sur la figure 6. Les trois premières étapes servent à éliminer le champignon et la dernière étape (ultrafiltration) sert à concentrer les enzymes produites.

Toutes les fractions de champignons récupérées (boues de décanteuse, crème de centrifugeuse et rétentat de microfiltration), sont placées dans le bioréacteur refroidi à 8°C pendant 72h et diluée avec de l'eau à un volume final égal à 4m3. Le moût subit une deuxième série de séparation: centrifugeuse, microfiltration et ultrafiltration.

La quantité d'enzymes récupérées à la fin de la deuxième série de séparation est analogue à celle obtenue à la fin de la première, soit environ 70 Kg de protéines après la première série de séparation et 64 Kg à la deuxième série de séparation. Par contre, il est intéressant de noter que l'activité β-glucosidase (figure 11) et l'activité FPase (figure 12) spécifiques du cocktail enzymatique obtenu sont de 20% supérieur après l'étape de refroidissement (autolyse du champignon). Le cocktail enzymatique récupéré est par conséquent beaucoup plus performant.

Des électrophorèses bidimensionnelles ont été réalisées pour voir s'il y avait une modification notable de la composition du cocktail enzymatique au cours des étapes de séparation. A partir d'échantillons préalablement dessalés à la FPLC [Fast protein liquid chromatography], avec des dépôts de 200µg sur le gel 2D, et coloration au bleu de Comassie, les protéines sont séparées selon leur masse moléculaire et leur point isoélectrique.
Les gels scannés sont présentés figure 13, la figure 13a correspond à la crème et la figure 13b au jus clair.
Les principales enzymes ont été identifiées sur l'étape de centrifugation dans le jus clair et la crème. On constate une différence notable sur les profils des gels. Les crèmes de centrifugation font apparaître des β-xylosidases. Cette activité a été mesurée sur pnp-xylose pour confirmation et on a trouvé une activité spécifique environ 3 fois supérieure dans les crèmes par rapport au cocktail enzymatique obtenu en fin de production (1,2 IU/mg sur les crèmes et 0,4 IU/mg sur le prélèvement final du fermenteur).

## Revendications

1. Procédé pour augmenter le taux de β-glucosidase et/ou de β-xylosidase d'un cocktail enzymatique à partir d'un microorganisme cellulolytique produisant des cellulases et/ou des hémi-cellulases, comprenant :
- une étape de production d'enzymes avec obtention d'un milieu contenant des enzymes et un moût de microorganisme, ledit moût est séparé ou non séparé du liquide contenant lesdites enzymes,
- une étape de refroidissement dudit moût à une température comprise entre 4 et 20°C, qui est une température inférieure à la température de l'étape de production d'enzymes et pendant un temps compris entre 12 et 90 h tel que :
- la concentration en β-glucosidase ou β-xylosidase du liquide issu de l'étape de refroidissement est supérieure à celle du liquide issu de l'étape de production d'enzymes,
et/ou
- le ratio du volume du solide/ volume total est inférieur audit ratio pour l'étape de production d'enzymes,
- et il est obtenu à l'issue de l'étape de refroidissement un cocktail enzymatique,
le microorganisme appartenant au genre *Trichoderma.*

2. Procédé selon la revendication 1, **caractérisé en ce que** le temps de l'étape de refroidissement est compris entre 24 et 72 h.

3. Procédé selon l'une des revendications précédentes dans lequel, à l'issue de l'étape de production d'enzymes, le moût est séparé du liquide.

4. Procédé selon la revendication précédente dans lequel ledit moût séparé est dilué par de l'eau.

5. Procédé selon la revendication 1 ou 2 dans lequel, à l'issue de la phase de production d'enzymes, le moût n'est pas séparé du liquide.

6. Procédé selon l'une des revendications précédentes dans lequel , après l'étape de refroidissement, le cocktail enzymatique issu du moût est séparé et il est obtenu un moût résiduel.

7. Procédé selon l'une des revendications 3 ou 4 et 6 dans lequel la séparation a lieu au moyen d'au moins une centrifugation ou filtration/pressage ou microfiltration, éventuellement précédée d'une décantation.

8. Procédé selon la revendication 6 dans lequel les enzymes du liquide séparé peuvent être concentrées, par exemple par ultrafiltration.

9. Procédé selon l'une des revendications précédentes dans lequel l'étape de refroidissement est réalisée à un pH compris entre 3,5 et 5,5.

10. Procédé selon l'une des revendications précédentes dans lequel l'étape de refroidissement est réalisée dans une atmosphère appauvrie en oxygène, de préférence dans une atmosphère anaérobie.

11. Procédé selon la revendication précédentes dans lequel il est injecté un gaz neutre, par exemple du gaz carbonique ou de l'azote.

12. Procédé selon l'une des revendications précédentes dans lequel le microorganisme appartient au genre *Trichoderma reesei,* et de préférence il s'agit de la souche CL847.

13. Procédé selon l'une des revendications précédentes dans lequel ledit moût résiduel est soumis à une étape de refroidissement, et il est obtenu à l'issue de l'étape de refroidissement un cocktail enzymatique, séparé ou non du moût résiduel, et de préférence ledit cocktail est séparé du moût résiduel de ladite étape de refroidissement.

14. Procédé selon l'une des revendications précédentes dans lequel les cocktails enzymatiques issus de moût sont mélangés.

15. Procédé selon l'une des revendications précédentes dans lequel au moins un cocktail enzymatique est mélangé au liquide séparé contenant les enzymes et issu de l'étape de production d'enzymes.

## Patentansprüche

1. Verfahren zur Erhöhung des Gehalts an β-Glucosidase und/oder β-Xylosidase eines Enzymcocktails aus einem Cellulose-Mikroorganismus, der Cellulasen und/oder Hemicellulasen produziert, umfassend:
- einen Schritt der Produktion von Enzymen, wobei ein Medium erhalten wird, das Enzyme und einen Schimmel eines Mikroorganismus enthält, wobei der Schimmel von der Flüssigkeit, welche die Enzyme enthält, getrennt wird oder nicht getrennt wird,
- einen Schritt des Abkühlens des Schimmels auf eine Temperatur zwischen 4 und 20 °C, die eine Temperatur unter der Temperatur des Schritts der Produktion von Enzymen ist, und während einer Zeit zwischen 12 und 90 h, so dass:
- die Konzentration von β-Glucosidase und/oder β-Xylosidase der Flüssigkeit aus dem Schritt des Abkühlens größer ist als jene der Flüssigkeit aus dem Schritt der Produktion von Enzymen, und/oder
- das Verhältnis des Volumens von Feststoff/Gesamtvolumen kleiner ist als das Verhältnis für den Schritt der Produktion von Enzymen,
- und am Ende des Schritts des Abkühlens ein Enzymcocktail erhalten wird, wobei der Mikroorganismus zur Gattung *Trichoderma* gehört.

2. Verfahren nach Anspruch 1, **dadurch gekennzeichnet, dass** die Zeit des Schritts des Abkühlens zwischen 24 und 72 h beträgt.

3. Verfahren nach einem der vorhergehenden Ansprüche, wobei, am Ende des Schritts der Produktion von Enzymen, der Schimmel von der Flüssigkeit getrennt wird.

4. Verfahren nach dem vorhergehenden Anspruch, wobei der abgetrennte Schimmel mit Wasser verdünnt wird.

5. Verfahren nach Anspruch 1 oder 2, wobei, am Ende der Phase der Produktion von Enzymen, der Schimmel nicht von der Flüssigkeit getrennt wird.

6. Verfahren nach einem der vorhergehenden Ansprüche, wobei, nach dem Schritt des Abkühlens, der Enzymcocktail, der von dem Schimmel stammt, getrennt wird, und ein verbleibender Schimmel erhalten wird.

7. Verfahren nach einem der Ansprüche 3 oder 4 und 6, wobei die Trennung mit Hilfe mindestens einer Zentrifugation oder Filtration/Pressung oder Mikrofiltration stattfindet, der gegebenenfalls eine Dekantierung vorausgeht.

8. Verfahren nach Anspruch 6, wobei die Enzyme der getrennten Flüssigkeit konzentriert werden können, zum Beispiel durch Ultrafiltration.

9. Verfahren nach einem der vorhergehenden Ansprüche, wobei der Schritt des Abkühlens bei einem pH zwischen 3,5 und 5,5 durchgeführt wird.

10. Verfahren nach einem der vorhergehenden Ansprüche, wobei der Schritt des Abkühlens in einer sauerstoffarmen Atmosphäre durchgeführt wird, vorzugsweise in einer anaeroben Atmosphäre.

11. Verfahren nach einem der vorhergehenden Ansprüche, wobei ein neutrales Gas injiziert wird, zum Beispiel Kohlendioxid oder Stickstoff.

12. Verfahren nach einem der vorhergehenden Ansprüche, wobei der Mikroorganismus zur Gattung *Trichoderma reesei* gehört, und es sich vorzugsweise um den Stamm CL847 handelt.

13. Verfahren nach einem der vorhergehenden Ansprüche, wobei der verbleibende Schimmel einem Schritt des Abkühlens unterzogen wird, und am Ende des Schritts des Abkühlens ein Enzymcocktail erhalten wird, der von dem verbleibenden Schimmel getrennt wird oder nicht, und vorzugsweise der Cocktail von dem verbleibenden Schimmel des Schritts des Abkühlens getrennt wird.

14. Verfahren nach einem der vorhergehenden Ansprüche, wobei die Enzymcocktails, die von dem Schimmel stammen, gemischt werden.

15. Verfahren nach einem der vorhergehenden Ansprüche, wobei mindestens ein Enzymcocktail mit der abgetrennten Flüssigkeit gemischt wird, welche die Enzyme enthält und aus dem Schritt der Produktion von Enzymen stammt.

## Claims

1. Process for increasing the β-glucosidase and/or de β-xylosidase rates of an enzymatic cocktail from a cellulolytic microorganism producing cellulases and/or hemi-cellulases, comprising:
- a step for the production of enzymes with a medium being obtained containing enzymes and a microorganism must, said must is separated, or not separated, from the liquid containing said enzymes,
- a step of cooling said must to a temperature comprised between 4 and 20°C, which is a temperature below the temperature of the enzyme production step, for a time comprised between 12 and 90 hours such that:
- the beta-glucosidase or β-xylosidase concentration of the liquid originating from the cooling step is greater than that of the liquid originating from the enzyme production step,
and/or
- the solid volume/total volume ratio is less than said ratio for the enzyme production step,
- and an enzymatic cocktail is obtained at the end of the cooling step.

2. Process according to claim 1 in which the duration of the cooling step is comprised between 24 and 72 hours.

3. Process according to any preceding claims in which, at the end of the enzyme production step, the must is separated from the liquid.

4. Process according to preceding claim in which said separated must is diluted with water.

5. Process according to claim 1 or 2 in which, at the end of the enzyme production phase, the must is not separated from the liquid.

6. Process according to one of the preceding claims in which, after the cooling step, the enzymatic cocktail originating from the must is separated and a residual must is obtained.

7. Process according to one of claims 3 or 4 and 6 in which the separation takes place by means of at least one centrifugation or filtration/pressing or microfiltration, optionally preceded by settling.

8. Process according to claim 6 in which the enzymes of the separated liquid can be concentrated, for example by ultrafiltration.

9. Process according to one of the preceding claims in which the cooling step is carried out at a pH comprised between 3.5 and 5.5.

10. Process according to one of the preceding claims in which the cooling step is carried out in an atmosphere depleted of oxygen, preferably in an anaerobic atmosphere.

11. Process according to preceding claim in which a neutral gas, for example carbon dioxide or nitrogen, is injected.

12. Process according to one of the preceding claims in which the microorganism belongs to the genus *Trichoderma,* in particular *Trichoderma reesei,* and is preferably the strain CL847.

13. Process according to one of the preceding claims in which said residual must is subjected to a cooling step and an enzymatic cocktail, separated or not separated from the residual must, is obtained at the end of the cooling step and preferably said enzymatic cocktail is separated from the residual must of said cooling step.

14. Process according to one of the preceding claims in which the enzymatic cocktails originating from must are mixed.

15. Process according to one of the preceding claims in which at least one enzymatic cocktail is mixed with the separated liquid containing the enzymes and originating from the enzyme production step.
